# EUROPEAN PATENT APPLICATION

(11) **EP 3 748 012 A1**
(43) Date of publication of application: **09.12.2020**
(21) Application number: 19746803.6
(22) Date of filing: 09.01.2019
(51) Int. Cl.: C12Q 1/6809, C12Q 1/04, C12Q 1/6881, C12N 5/071, C12N 5/0735, C12N 15/113

(54) **METHOD FOR EVALUATING STATE OF UNDIFFERENTIATED CELL AND UTILIZATION THEREOF**

(30) Priority: 30.01.2018 JP 2018014024
(71) Applicant: SYSMEX CORPORATION, Kobe-shi Hyogo 651-0073 (JP)
(72) Inventor: OKA Yuma, Kobe-shi, Hyogo 651-0073 (JP); AIHARA Yuki, Kobe-shi, Hyogo 651-0073 (JP)
(74) Representative: De Clercq & Partners
(86) International application number: PCT/JP2019/000305
(87) International publication number: WO 2019/150891

(57) **Abstract**

A problem to be addressed is to provide a method that can evaluate the state of undifferentiated cells without destroying cells. The above problem is solved by providing a method for evaluating the state of undifferentiated cells. The method includes the steps of: measuring at least one miRNA selected from miR371, miR372, and miR373 in a liquid phase fraction of a liquid that contains undifferentiated cells; and evaluating a state of the undifferentiated cells on the basis of a measurement value of the miRNA.

## Description

### TECHNICAL FIELD

The present invention relates to a method for evaluating the state of undifferentiated cells. The present invention relates to a reagent kit. The present invention relates to a detection method of an amplification product. The present invention relates to a method for monitoring an induction operation of undifferentiated cells.

### BACKGROUND ART

Pluripotent stem cells are expected to be a cell source that plays an important role for realizing regenerative medicine such as cell transplantation. However, stem cell lines established to date are not uniform cell populations having different degrees of undifferentiation, and differentiation characteristics and pluripotency are different among the lines. For example, conventional human embryonic stem cells (ES cells) include not only undifferentiated cells at a stage of preimplantation embryo but also undifferentiated cells at various developmental stages. Conventional human induced pluripotent stem cells (iPS cells) are also known to be not uniform cell populations reprogrammed at various developmental stages. Thus, even when differentiation of pluripotent stem cells is induced by using the same protocol, efficiency in the differentiation induction varies to a great extent.

In recent years, classifying pluripotent stem cells into a naive state type and a primed state type has been proposed. Naive state pluripotent stem cells are known to be cells that have characteristics corresponding to preimplantation embryo. Naive state pluripotent stem cells are uniform and can maintain a stable ground state. Primed state pluripotent stem cells are known to be cells that have characteristics corresponding to postimplantation embryo and are cells at advanced developmental stages compared with naive state pluripotent stem cells. Naive state pluripotent stem cells have higher replication competence and higher pluripotency than primed state pluripotent stem cells, and thus, are attracting attention in the field of regenerative medicine. Therefore, it is desired to establish a stem cell line having the characteristics of the naive state type with the ground state thereof maintained. In recent years, methods for establishing human stem cells that are highly uniform and that indicate the phenotype of the naive state type with the ground state maintained, have been successively reported.

As described above, naive state human stem cells are expected to be applied to regenerative medicine. However, if the population of stem cells do not uniformly have the phenotype of the naive state type, efficiency in differentiation induction varies. In order to use high-quality undifferentiated cells in regenerative medicine, means for evaluating the state of undifferentiated cells is required. At present, expression analysis of naive-related genes, and the like, exist as methods for evaluating naive state pluripotent stem cells. For example, NON PATENT LITERATURE 1 discloses that, in naive state pluripotent stem cells, transcription factor TFE3 is localized in the nucleus. NON PATENT LITERATURE 2 discloses that, in naive state pluripotent stem cells, the intracellular expression level of a miR371-373 cluster of microRNA (miRNA) is higher than that of primed state pluripotent stem cells. However, these expression analyses are destructive inspections, and valuable undifferentiated cells are consumed for quality evaluation.

As an example of nondestructive inspection, discerning the primed state type and the naive state type on the basis of difference in the morphology of cell colonies is known. However, observing all the cell samples with a microscope requires tremendous work. In addition, such an inspection is based on observation of the morphology of cells, and thus, is not objective. NON PATENT LITERATURE 3 discloses evaluating pluripotency of pluripotent stem cells by measuring the amount of miRNA in a culture supernatant of the cells. However, the miRNA to be detected in NON PATENT LITERATURE 3 is a miRNA that is known to exist only in mice. In addition, in NON PATENT LITERATURE 3, discerning the naive state type and the primed state type of human pluripotent stem cells on the basis of the amount of miRNA has not been considered.

### CITATION LIST

### [NON PATENT LITERATURE]

[NPL 1] Gafni O. et al., Derivation of novel human ground state naive pluripotent stem cells, Nature, 2013, vol.504, p.282-286
[NPL 2] Rosa A. et al., Regulatory non-coding RNAs in pluripotent stem cells, Int. J. Mol. Sci., 2013, vol.14, p. 14346-14373
[NPL 3] Zhang Y. et al., A non-invasive method to determine the pluripotent status of stem cells by culture medium microRNA expression detection, Sci. Rep., 2016, 6:22380

### SUMMARY OF THE INVENTION

### PROBLEMS TO BE SOLVED BY THE INVENTION

An object of the present invention is to provide a method that can evaluate the state of undifferentiated cells without destroying cells. The present inventors have newly found that undifferentiated cells of which the developmental stage is closer to that of early embryo release larger amounts of miR371, miR372, and miR373 to the outside of the cells, than undifferentiated cells of which the developmental stage is more advanced. Accordingly, the present inventors have found that the state of undifferentiated cells can be evaluated by measuring miR371, miR372, and miR373 in the liquid phase fraction of a cell culture solution, and have completed the present invention.

### SOLUTION TO THE PROBLEMS

The present invention provides a method for evaluating a state of undifferentiated cells. The method includes the steps of: measuring at least one miRNA selected from miR371, miR372, and miR373 in a liquid phase fraction of a liquid that contains undifferentiated cells; and evaluating a state of the undifferentiated cells on the basis of a measurement value of the miRNA.

The present invention provides a reagent kit to be used in the method above. The reagent kit includes at least one primer capable of hybridizing to at least one miRNA selected from miR371, miR372, and miR373.

The present invention provides a detection method of an amplification product. The detection method includes the steps of: obtaining a liquid phase fraction of a liquid comprising undifferentiated cells; mixing at least one miRNA selected from miR371, miR372, and miR373 contained in the liquid phase fraction, a primer, and a polymerase together, to prepare a reaction solution; and amplifying DNA fragments including cDNA of the miRNA in the reaction solution and detecting an amplification product.

The present invention provides a method for monitoring an induction operation of undifferentiated cells. The method includes: obtaining a liquid phase fraction of a liquid comprising undifferentiated cells, before an induction operation from primed state undifferentiated cells into naive state undifferentiated cells, and obtaining a first measurement value of at least one miRNA selected from miR371, miR372, and miR373 in the liquid phase fraction; obtaining a liquid phase fraction of the liquid comprising undifferentiated cells, during the induction operation from primed state undifferentiated cells into naive state undifferentiated cells, and obtaining a second measurement value of at least one miRNA selected from miR371, miR372, and miR373 in the liquid phase fraction; and comparing the first measurement value and the second measurement value with each other, thereby monitoring the induction operation of the undifferentiated cells.

The present invention provides a method for monitoring an induction operation of undifferentiated cells. The method includes: obtaining a liquid phase fraction of a liquid comprising undifferentiated cells, at a first time point during an induction operation from primed state undifferentiated cells into naive state undifferentiated cells, and obtaining a first measurement value of at least one miRNA selected from miR371, miR372, and miR373 in the liquid phase fraction; obtaining a liquid phase fraction of the liquid comprising undifferentiated cells, at a second time point during the induction operation from primed state undifferentiated cells into naive state undifferentiated cells, and obtaining a second measurement value of at least one miRNA selected from miR371, miR372, and miR373 in the liquid phase fraction; and comparing the first measurement value and the second measurement value with each other, thereby monitoring the induction operation of the undifferentiated cells.

The present invention provides a reagent kit including at least one primer selected from: a primer having a base sequence indicated in (1) below on a 3' end side; a primer having a base sequence indicated in (2) below on a 3' end side; a primer having a base sequence indicated in (3) below on a 3' end side; and a primer having a base sequence indicated in (4) below on a 3' end side.

5'-ACACT-3' ··· (1)

5'-GCACT-3' ··· (2)

5'-ACGCT-3' ··· (3)

5'-ACACC-3' ··· (4)

The present invention provides a mixed liquid including: a liquid phase fraction of a cell culture solution of undifferentiated cells; a reverse transcriptase; a primer capable of hybridizing to at least one miRNA selected from miR371, miR372, and miR373; and dNTPs.

### ADVANTAGEOUS EFFECTS OF THE INVENTION

The present invention enables evaluating the state of undifferentiated cells without destroying cells.

### BRIEF DESCRIPTION OF THE DRAWINGS

[FIG. 1A] FIG. 1A is a bright field image of primed state iPS cells prepared in Example 1.
[FIG. 1B] FIG. 1B is a bright field image of naive state iPS cells prepared in Example 1.
[FIG. 2] FIG. 2 shows a bright field image and immunostaining images of cells having TFE3 localized in the nucleus.
[FIG. 3] FIG. 3 is a graph indicating the proportion of cells having TFE3 localized in the nuclei, for each of naive state iPS cells and primed state iPS cells.
[FIG. 4] FIG. 4 is a graph indicating, for each of miR371, miR372, and miR373, the relative expression level in a culture supernatant of naive state iPS cells, with the expression level in a culture supernatant of primed state iPS cells assumed to be 1.

### DESCRIPTION OF EMBODIMENTS

### [1. Undifferentiated cell evaluation method]

In an undifferentiated cell evaluation method of the present embodiment (hereinafter, also simply referred to as "method"), at least one miRNA selected from miR371, miR372, and miR373 in a liquid phase fraction of a liquid that contains undifferentiated cells, is measured.

A specimen used in the method of the present embodiment is a liquid phase fraction of a liquid that contains undifferentiated cells. The "liquid that contains undifferentiated cells" means a liquid that is in a state of being in contact with undifferentiated cells or having undifferentiated cells immersed therein in a culture container, and that is capable of holding undifferentiated cells viable. In the liquid that contains undifferentiated cells, the undifferentiated cells may be suspended in the liquid capable of holding undifferentiated cells viable, or they may sediment or be attached to a bottom part of a culture container, in the liquid.

The liquid capable of holding undifferentiated cells viable is not limited in particular as long as the liquid does not kill or significantly damage the undifferentiated cells for a predetermined period. Examples of such a liquid include a liquid medium for undifferentiated cells, a saline solution, and a buffer solution (e.g., PBS, HEPES). Among these, the liquid medium for undifferentiated cells is preferable. In this specification, when a liquid medium is used as the liquid capable of holding undifferentiated cells viable, the liquid that contains undifferentiated cells is also referred to as a "cell culture solution of undifferentiated cells".

In this specification, a "liquid phase fraction" is the entirety or part of the solution portion of the liquid that contains undifferentiated cells, and substantially does not contain cells. The liquid phase fraction of the liquid that contains undifferentiated cells is preferably a culture supernatant. The culture supernatant is a liquid phase fraction of the cell culture solution of the undifferentiated cells.

The liquid medium for undifferentiated cells can be selected as appropriate from known liquid media in accordance with the type of the undifferentiated cells. Examples of such a liquid medium include IMDM medium, Medium 199 medium, EMEM medium, αMEM medium, DMEM medium, Ham's F12 medium, RPMI 1640 medium, Fischer's medium, MEF-CM, StemPro (trademark) 34 (Invitrogen), Essential 8 (trademark) (Thermo Fisher Scientific Inc.), hPSC Growth Medium DXF (trademark) (Takara Bio Inc.), StemFit (trademark) AK02N (Takara Bio Inc.), ReproNaive (trademark) (ReproCELL Inc.), and a mixed medium of these.

The culture medium may contain components necessary for proliferation and maintenance of cells. Examples of the components include serum, albumin, transferrin, alternative serum (Knockout (trademark) Serum Replacement) (KSR), N2 supplement (Invitrogen), B27 supplement (Invitorogen), fatty acid, insulin, collagen precursor, 2-mercaptoethanol, thiol glycerol, lipid, amino acid, L-glutamine, Glutamax (trademark) (Invitorogen), nonessential amino acid, vitamin, proliferation factor, antibiotic, antioxidant, pyruvic acid, buffer agent, and inorganic salt.

When undifferentiated cells are pluripotent stem cells, components necessary for maintenance of viability or the undifferentiated state thereof may be added to the culture medium. Examples of such components include leukemia inhibitory factor (LIF), activin, fibroblast growth factor (FGF), stem cell factor, MEK inhibitor, GSK3 inhibitor, and ROCK inhibitor.

In the present embodiment, undifferentiated cells are not limited in particular as long as the undifferentiated cells are cells that have differentiation potency. Examples of the undifferentiated cells include stem cells and progenitor cells. As the stem cells, there are pluripotent stem cells and somatic stem cells. Pluripotent stem cells are stem cells that have a capability (pluripotency) of differentiating into cells derived from ectoderm, endoderm, and mesoderm, and that have proliferation potency. Examples of the pluripotent stem cells include induced pluripotent stem cells (iPS cells), embryonic stem cells (ES cells), nuclear transfer ES cells (ntES cells), and embryonic germ cells (EG cells).

An iPS cell is produced by introducing a predetermined reprogramming factor (DNA or protein) such as Oct3/4, Klf4, c-Myc, or Sox2 into a somatic cell such as a skin cell, and is a stem cell having pluripotency and proliferation potency. An ES cell is a stem cell derived from an inner cell mass of a blastocyst of a mammal, and is a stem cell having pluripotency and proliferation potency. An ntES cell is an ES cell established from an inner cell mass of a blastocyst derived from a cloned embryo obtained by substituting the nucleus of an unfertilized egg with the nucleus of a somatic cell. An ntES cell has substantially the same characteristics as an ES cell. An EG cell is a cell established from a primordial germ cell at an embryonic stage, and has pluripotency similar to that of an ES cell. An EG cell can be established by culturing a primordial germ cell in the presence of a substance such as LIF, basic FGF (bFGF), or stem cell factor.

Somatic stem cells exist in various tissues, and are stem cells that supply new cells in the tissues. Differentiation potency of somatic stem cells are limited when compared with that of pluripotent stem cells. Examples of the somatic stem cells include mesenchymal stem cells, neural stem cells, epithelial stem cells, hepatic stem cells, germline stem cells, hematopoietic stem cell, and skeletal muscle stem cells.

A progenitor cell is a cell that develops from a stem cell and differentiates into a terminally differentiated cell. The terminally differentiated cell means a cell that does not have differentiation potency and that has reached a terminal differentiation in an embryological cell lineage. Examples of progenitor cells include proplatelets, liver progenitor cells, heart progenitor cells, and neural progenitor cells.

The undifferentiated cells are preferably pluripotent stem cells, and more preferably, iPS cells, ES cells, ntES cells, or EG cells. In the present embodiment, the undifferentiated cells may be cells in an intermediate stage where cells not having differentiation potency are induced into cells having differentiation potency. Examples of such cells include somatic cells into which a predetermined reprogramming factor has been introduced in order to produce iPS cells.

The undifferentiated cells may be undifferentiated cells collected from a living organism, or may be cells that have been artificially prepared by reprogramming differentiated cells collected from a living organism. From the viewpoint of regenerative medicine research and clinical application, pluripotent stem cells artificially prepared are preferable. The origin of the undifferentiated cells is not limited in particular. The undifferentiated cells are preferably derived from a mammal such as human, monkey, dog, cat, horse, mouse, rat, hamster, guinea pig, rabbit, sheep, pig, or cattle.

In recent years, classifying pluripotent stem cells into a naive state type and a primed state type has been proposed. "Naive state pluripotent stem cells" means pluripotent stem cells of which the developmental stage corresponds to that of preimplantation embryo. "Primed state pluripotent stem cells" means pluripotent stem cells of which the developmental stage corresponds to that of postimplantation embryo. In general, naive state pluripotent stem cells are known to be cells that maintain a uniform ground state and that exhibit high pluripotency, when compared with primed state pluripotent stem cells. Known characteristics of naive state pluripotent stem cells and primed state pluripotent stem cells are as follows.

### <Characteristics of naive state pluripotent stem cells>

- Cell colonies are small and have dome-like morphology
- A wide range of DNA demethylation over the entirety of genome
- X chromosome is in activated state (XaXa)
- High proportion of predetermined transcription factor (for example, TFE3) being localized in nucleus.

### <Characteristics of primed state pluripotent stem cell>

- Cell colonies are large and have flat morphology
- DNA methylation
- X chromosome is in inactivated state (XaXi)
- Low proportion of predetermined transcription factor (for example, TFE3) being localized in nucleus.

In the case of mouse ES cells, naive state ES cells can be obtained by culturing cells in a chemical synthesis medium 2i/LIF medium that contains LIF, an MEK inhibitor, and a GSK3 inhibitor. Meanwhile, with a conventional culture method, human ES cells and human iPS cells exhibit characteristics corresponding to those of postimplantation embryo, and are classified into the primed state type. In recent years, a method in which NANOG gene and KLF2 gene are introduced into primed state human pluripotent stem cells, thereby inducing the cells into the naive state type, has been reported (see Takashima Y. et al., Cell, vol.158, p 1254-1269, 2014). A liquid medium that allows induction from the primed state type into the naive state type without performing gene introduction (hereinafter, also referred to as "induction medium") is also known. An example of such an induction medium is ReproNaive (trademark) (ReproCELL Inc.).

In one embodiment, the undifferentiated cells are primed state pluripotent stem cells, naive state pluripotent stem cells, or a mixture thereof, but are not known to be which of them. In another embodiment, the undifferentiated cells are cells of which the type has been identified, the primed state type or the naive state type, by a method different from the method of the present invention. In this case, which of the primed state pluripotent stem cells and the naive state pluripotent stem cells the cells are can be re-verified by the method of the present embodiment. The undifferentiated cells may be pluripotent cells in an intermediate stage in which the cells are being induced from the primed state type into the naive state type. Examples of such cells include: pluripotent stem cells into which NANOG gene and KLF2 gene have been introduced; and pluripotent stem cells for which a liquid medium has been substituted with an induction medium.

The culture method for the undifferentiated cells can be selected as appropriate from known culture methods in accordance with the type of the undifferentiated cells. For cell culture, usually, suspension culture, adhesion culture, or a combination thereof is used. The suspension culture is a culture that is performed under a condition that cells are non-adhesive to a culture container. In the suspension culture, cells may not necessarily be dispersed in the liquid medium, and may sediment on a bottom part of the culture container. The culture container to be used for the suspension culture is not limited in particular. Examples of the culture container include a flask, a dish, a plate, a chamber, and a tube. The culture container to be used for the suspension culture is preferably non-cell-adhesive. The culture container is preferably formed of a hydrophobic material, or coated with a coating agent (e.g., polyhydroxyethyl methacrylate copolymer) that prevents adhesion of cells to the surface thereof.

The adhesion culture is a culture in which cells are adhered to a support body. A culture container is used as the support body. The culture container to be used for the adhesion culture is not limited in particular. For the adhesion culture, the same type of culture container as that used in the suspension culture can be used. However, the surface or inside of the culture container is preferably coated with a coating agent (also referred to as "cell culture substrate", "adhesion substrate", or the like) that causes cells to be adhered thereto. Examples of the coating agent include gelatin, laminin, collagen, Poly-D-Lysine, polyornithine, fibronectin, and vitronectin. A commercial product such as iMatrix-511 (Nippi Inc.) containing Laminin 511-E8 fragment can also be used. A known scaffold can also be used as the support body. By using this, undifferentiated cells can proliferate on the scaffold, thereby realizing three-dimensional culture. A commercial product can be used as the scaffold. Examples of the scaffold include: Matrigel (trademark) (Corning); QGel (trademark) MT 3D Matrix (Qgel SA); 3-D Life Biomimetic (Cellendes); Puramatrix (3D MATRIX); and alvetex (Reinnavate).

The undifferentiated cell may be cultured together with feeder cells, if necessary. The type of the feeder cells is not limited in particular. The feeder cells can be selected from known feeder cells as appropriate. Examples of the feeder cells include mouse embryonic fibroblasts (MEF), a mouse embryonic fibroblast line (STO), SL10 cells, and SNL cells.

The culture condition can be adjusted as appropriate in accordance with the type of the undifferentiated cells. For example, the culture temperature is preferably about 30 to 40°C, and the carbon dioxide concentration is preferably about 1 to 10%.

In the method of the present embodiment, a liquid phase fraction of a liquid that contains undifferentiated cells is collected as a specimen for miRNA measurement. The method for obtaining the liquid phase fraction from the liquid that contains undifferentiated cells is not limited in particular. As the method for obtaining the liquid phase fraction, in the case of the suspension culture, the liquid that contains undifferentiated cells is centrifuged, whereby a centrifuged supernatant can be obtained as the liquid phase fraction of the present embodiment. Since cells sediment in a liquid, the cells may be caused to be dispersed therein, and then, a liquid phase fraction may be obtained from a lower portion of the culture container. The liquid that contains undifferentiated cells may be filtered to be separated into a fraction that contains cells and a liquid phase fraction, whereby the liquid phase fraction may be obtained.

In the case of the adhesion culture, cells are adhered to a bottom part of the culture container. Thus, the liquid phase fraction of the present embodiment can be obtained by suctioning the supernatant in the culture container with a pipette or the like.

When there is a possibility that cells are mixed in the obtained liquid phase fraction, the cells may be separated/removed by subjecting the liquid phase fraction to centrifugation, filtration, or the like.

The timing when the liquid phase fraction is obtained from the liquid that contains undifferentiated cells is not limited in particular. After an undifferentiated cell is seeded in a culture container containing a liquid capable of holding undifferentiated cells viable, preferably, a liquid medium, the liquid phase fraction can be collected at an arbitrary time point. Alternatively, the liquid phase fraction may be collected after the medium has been replaced. For example, the liquid medium is removed from the undifferentiated cells cultured in the culture container, and after 1 to 168 hours after the medium has been replaced with a liquid capable of holding undifferentiated cells viable, the liquid phase fraction may be collected. Alternatively, undifferentiated cells cultured in advance are transferred to another culture container containing a liquid capable of holding undifferentiated cells viable, and then, after 1 to 168 hours, the liquid phase fraction may be collected.

miRNAs to be measured in the present embodiment are miR371, miR372, and miR373. miR371 forms a family including miR371a and miR371b. A gene group that codes human miR371, miR372, and miR373 exists at chromosome 19, and forms a miR371-373 cluster. In this specification, "miRNA in a miR371-373 cluster" means miRNAs transcribed from these genes. Hereinafter, "to measure at least one miRNA selected from miR371, miR372, and miR373" is also referred to as "to measure miRNA in a miR371-373 cluster".

After being transcribed from the gene, each miRNA matures through pri-miRNA and pre-miRNA. In the present embodiment, it is preferable to measure mature miRNA. Nucleotide sequences of mature miRNAs of miR371a, miR371b, miR372, and miR373 are indicated at SEQ ID NOs. 1 to 4. Genes that code these miRNAs are disclosed as in Table 1 in the GenBank database.

**[Table 1]**

| Gene | GenBank Accession No. |
|---|---|
| miR371a gene | NR_029864.1 |
| miR371b gene | NR_039909.1 |
| miR372 gene | NR_029865.1 |
| miR373 gene | NR_029866.1 |

The method for measuring miRNA is not limited in particular, and a known method can be used. Examples of the measurement method include: a measurement method using hybridization of a probe; a measurement method using a nucleic acid amplification technique; a measurement method using mass spectrometry; and a measurement method using sequencing. Examples of the measurement method using hybridization of a probe include: a microarray method; a northern hybridization method; and an RNase protection assay. Examples of the measurement method using a nucleic acid amplification technique include a quantitative RT-PCR method and a quantitative RT-LAMP method. Examples of the quantitative RT-PCR method include the SYBR (trademark) Green method and the TaqMan (trademark) method. Since a miRNA to be amplified is short, when a method using nucleic acid amplification is used, a stem-loop primer, poly(A) addition, or the like is usually used.

In the method of the present embodiment, the state of the undifferentiated cells is evaluated on the basis of a measurement value of miRNA. The measurement value of miRNA may be any of a measurement value of miR371a, a measurement value of miR371b, a measurement value of miR372, and a measurement value of miR373, or a sum of at least two of these measurement values. The measurement value of miR371 may be a measurement value of miR371a, a measurement value of miR371b, or a sum of these.

In this specification, a "measurement value" is a value that reflects the amount of miRNA existing in a liquid phase fraction. Examples of the measurement value include: an optical measurement value (fluorescence intensity, turbidity, absorbance, etc.); a reaction cycle number or reaction time when the optical measurement value reaches a predetermined reference value; and a quantitative value (copy number, mass, or concentration) of miRNA calculated using a calibration curve. The predetermined reference value can be set as appropriate in accordance with the type of the value indicating the existing amount or concentration of the miRNA. For example, when a quantitative RT-PCR method is used, an amount of change in the fluorescence intensity when nucleic acid amplification reaction indicates logarithmic amplification can be set, with a predetermined cycle number used as a reference value.

In pluripotent stem cells, since miR371, miR372, and miR373 bind to specific mRNAs in cells and have a function of inhibiting translation to protein, the presence of miR371, miR372, and miR373 in cells is known. However, it is not known that miR371, miR372, and miR373 are released to the outside of pluripotent stem cells. Surprisingly, the present inventors have found that miR371, miR372, and miR373 are released to the outside of pluripotent stem cells. In particular, the present inventors have found that, in the case of naive state pluripotent stem cells, larger amounts of miR371, miR372, and miR373 are released to the outside of the cells than in the case of primed state pluripotent stem cells. Therefore, in the present embodiment, when the undifferentiated cells are pluripotent stem cells, the measurement value of miRNA can be used as an index for existence of naive state pluripotent stem cells in a liquid that contains undifferentiated cells.

An induction operation from primed state undifferentiated cells into naive state undifferentiated cells means a predetermined process, performed on primed state undifferentiated cells, for inducing primed state undifferentiated cells into naive state undifferentiated cells. Examples of such a process include the above-described gene introduction and cell culture thereafter, and culturing in an induction medium. "During an(the) induction operation" means a time period after the start and before the completion of the induction operation.

As described above, the developmental stage of naive state pluripotent stem cells is closer to that of early embryo, than the developmental stage of primed state pluripotent stem cells is. Thus, naive state pluripotent stem cells are considered to be useful in regenerative medicine. Usually, it takes several hours to several weeks before completion of the induction, i.e., before naive state undifferentiated cells can be obtained from primed state undifferentiated cells. In the method of the present embodiment, since the above-described measurement value of miRNA serves as an index for existence of naive state pluripotent stem cells, the state of pluripotent stem cells during the induction operation can be monitored. In this case, a liquid phase fraction is collected from a cell culture solution of pluripotent stem cells during the induction operation from the primed state type into the naive state type, and the liquid phase fraction is used in miRNA measurement.

For example, miRNA in the liquid phase fraction is measured before the start of and during the induction operation from primed state undifferentiated cells into naive state undifferentiated cells, and the obtained two measurement values are compared with each other. When there is no big difference between the measurement value of miRNA before the start of the induction operation and the measurement value of miRNA during the induction operation, it is determined that the induction operation is not being appropriately performed, and the induction operation can be interrupted. When the measurement value of miRNA during the induction operation is greater than the measurement value of miRNA before the start of the induction operation, it is determined that the induction operation is being appropriately performed, and the induction operation can be continued. Alternatively, it may be determined that primed state pluripotent stem cells have been induced into naive state pluripotent stem cells, and the induction operation may be completed.

In another example, miRNA in a miR371-373 cluster is measured at a plurality of time points during an induction operation, and the measurement values are compared with each other, whereby the progress state of the induction into the naive state type can be monitored. Specifically, first, at a first time point during the induction operation into the naive state type, a liquid phase fraction is collected from a cell culture solution, and miRNA in a miR371-373 cluster is measured to obtain a first measurement value. Next, at a second time point during the induction operation into the naive state type, a liquid phase fraction is collected from the cell culture solution, and miRNA in a miR371-373 cluster is measured to obtain a second measurement value. Then, the first measurement value and the second measurement value are compared with each other. Accordingly, the progress state of the induction can be evaluated. For example, when the second measurement value is greater than the first measurement value, it is determined that the induction is being appropriately performed, and the induction operation can be continued. Alternatively, it may be determined that the primed state type has been induced into the naive state type, and the induction operation may be completed.

In still another embodiment, a liquid phase fraction of a liquid that contains pluripotent stem cells after an induction operation into the naive state type has been completed is used in miRNA measurement. Even when the induction operation itself has been completed, all of the pluripotent stem cells have not necessarily become naive state pluripotent stem cells, and there is a possibility that primed state pluripotent stem cells remain. As described above, in a cell population where primed state pluripotent stem cells and naive state pluripotent stem cells are mixed, efficiency in differentiation induction varies. Thus, the quality of the cell population to serve as pluripotent stem cells to be used in regenerative medicine is reduced. Thus, as quality inspection of the cell population after the completion of the induction operation, whether the pluripotent stem cells are of the naive state type or have the primed state type mixed therein is determined on the basis of the measurement value of miRNA. This determination result can be used in evaluation of the quality of pluripotent stem cells, for example. The determination can be performed through comparison between the measurement value of miRNA and a predetermined threshold. For example, when the measurement value of miRNA is greater than or equal to a first threshold, it can be determined that the undifferentiated cells are naive state pluripotent stem cells. In this case, it can be determined that the induced pluripotent stem cells can be used in regenerative medicine or research therefor. When the measurement value of miRNA is smaller than the first threshold, it can be determined that primed state pluripotent stem cells are mixed in the undifferentiated cells. In this case, it is possible to determine to further perform the induction operation, for example.

In still another embodiment, the state of pluripotent stem cells can be evaluated during or after completion of an induction from the primed state type into the naive state type. In this case, monitoring of the progress state of the induction and quality inspection of induced pluripotent stem cells can be performed.

In another embodiment, whether the undifferentiated cells are of the naive state type or the primed state type can be determined by a combination of the method of the present embodiment and another method. For example, the determination can be performed by comprehensively taking the morphology of colonies, localization of TFE3 in the nucleus, the method of the present embodiment, and the like into consideration. In another example, the determination result obtained by a method based on the morphology of colonies, localization of TFE3 in the nucleus, and the like can be validated by the method of the present embodiment.

In any of the embodiments above, when the medium is replaced with a new medium at the start of the induction from the primed state type into the naive state type, the old medium may be used in miRNA measurement.

Depending on the purpose of a test, a research, or the like, primed state pluripotent stem cells may be required. In still another embodiment, whether or not pluripotent stem cells are of the primed state type is determined on the basis of a measurement value of miRNA. The determination can be performed through comparison between the measurement value of miRNA and a predetermined threshold. In the case of primed state pluripotent stem cells, the amount of miR371, miR372, and miR373 to be released to the outside of the cells is smaller than in the case of naive state pluripotent stem cells. Therefore, the predetermined threshold is preferably lower than the above-described first threshold. Here, the predetermined threshold lower than the first threshold is referred to as a second threshold. When the measurement value of miRNA is smaller than the second threshold, it can be determined that the undifferentiated cells are primed state pluripotent stem cells. When the measurement value of miRNA is greater than or equal to the second threshold, it can be determined that naive state pluripotent stem cells are mixed in the undifferentiated cells.

Preferably, the predetermined thresholds are set in advance before performing the method of the present embodiment. For example, a liquid phase fraction of a cell culture solution that contains naive state pluripotent stem cells only; a liquid phase fraction of a cell culture solution that contains pluripotent stem cells during an induction operation from the primed state type into the naive state type; and a liquid phase fraction of a cell culture solution that contains primed state pluripotent stem cells only, are each prepared in a plural number. Then, miRNA in a miR371-373 cluster is measured for each liquid phase fraction. Then, a value that can most accurately classify the cell culture solution containing naive state pluripotent stem cells only can be set as the first threshold. Similarly, a value that can most accurately classify the cell culture solution containing primed state pluripotent stem cells only can be set as the second threshold.

The present embodiment includes semi-quantitatively detecting the existing amount of naive state pluripotent stem cells. The semi-quantitative detection means indicating the existing amount of pluripotent stem cells in a stepwise manner such as "-", "+", "++" (negative, weakly positive, strongly positive). The semi-quantitative detection of naive state pluripotent stem cells can be performed through comparison between a measurement value of miRNA and a plurality of thresholds. As the plurality of thresholds, the first threshold and the second threshold described above can be used, for example. Specific determinations are as follows. When the measurement value of miRNA is smaller than the second threshold, "-" indicating that naive state pluripotent stem cells do not exist can be determined. When the measurement value of miRNA is greater than or equal to the second threshold and smaller than the first threshold, "+" indicating that a small amount of naive state pluripotent stem cells exist can be determined. When the measurement value of miRNA is greater than or equal to the first threshold, "++" indicating that naive state pluripotent stem cells exist can be determined.

### [2. Reagent kit]

The scope of the present invention includes a reagent kit to be used in the method for evaluating the state of undifferentiated cells according to the present embodiment described above. The reagent kit of the present embodiment includes at least one primer capable of hybridizing to at least one miRNA selected from miR371, miR372, and miR373. The primer may be a primer for reverse transcription of the above miRNA (hereinafter, also referred to as "RT primer"), or may be a forward primer and/or a reverse primer for amplifying cDNA synthesized from the above miRNA (hereinafter, also referred to as "cDNA amplification primer"). The reagent kit of the present embodiment may include both of the RT primer and the cDNA amplification primer.

When mature miRNA is measured by using the primer, since mature miRNA has a short nucleotide length, a stem-loop primer can be used as the reverse transcription primer and/or the cDNA amplification primer. The above-described primer may be bound to a solid phase such as a plate or particles.

The scope of the present invention also includes a reagent kit that includes at least one primer selected from: a primer having a base sequence indicated in (1) below on the 3' end side; a primer having a base sequence indicated in (2) below on the 3' end side; a primer having a base sequence indicated in (3) below on the 3' end side; and a primer having a base sequence indicated in (4) below on the 3' end side.

5'-ACACT-3' ··· (1)

5'-GCACT-3' ··· (2)

5'-ACGCT-3' ··· (3)

5'-ACACC-3' ··· (4)

This reagent kit may be used in the method for evaluating the state of undifferentiated cells of the present embodiment described above. This reagent kit may be used in a detection method of an amplification product described later.

The base sequences of (1) to (4) above are sequences complementary to five bases from the 3' end of the respective base sequences of SEQ ID NOs. 1 to 4. The above primers have these base sequences on the 3' end side, and thus can hybridize to the respective mature miRNAs of miR371a, miR371b, miR372, and miR373. Then, the 3' end of the primer is elongated by a polymerase, and a complementary strand on the 5' end side of the mature miRNA is synthesized. In these primers, arbitrary base sequences may be added on the 5' end side of the base sequences of (1) to (4) above.

Each primer may be an RT primer or a cDNA amplification primer. As to the form, each primer may be a linear primer or a stem-loop primer. Each primer may be bound to a solid phase such as a plate or particles.

Each of the reagent kits above may include other reagents such as a buffer solution, dNTPs (dATP, dTTP, dGTP, and dCTP), and a polymerase.

### [3. Detection method of amplification product]

The scope of the present invention also includes a method for detecting (hereinafter, also referred to as "detection method") an amplification product derived from at least one miRNA selected from miR371, miR372, and miR373 contained in a liquid phase fraction. In the detection method of the present embodiment, first, a liquid phase fraction of a liquid that contains undifferentiated cells is obtained. Details of undifferentiated cells, a liquid containing the cells, a liquid phase fraction of the liquid, and obtaining of the liquid phase fraction are the same as those described with respect to the method of the present embodiment above.

Next, at least one miRNA selected from miR371, miR372, and miR373 contained in the liquid phase fraction, a primer, and a polymerase are mixed together to prepare a reaction solution. In the present embodiment, miRNA is preferably extracted from the liquid phase fraction. miRNA can be extracted by a known method. For example, Total RNA including miRNA can be collected by subjecting the liquid phase fraction to phenol/chloroform extraction. Extraction and purification of miRNA from the liquid phase fraction can also be performed by using a commercial kit.

Examples of the primer include a primer for reverse transcription, and a cDNA amplification primer. When synthesis of cDNA from miRNA and amplification of cDNA are performed in two steps, i.e., sequentially performed, the primer for reverse transcription may be a random primer, or may be a primer capable of hybridizing to each mature miRNA of miR371, miR372, and miR373. When synthesis of cDNA from miRNA and amplification of cDNA is performed in one step, i.e., simultaneously performed, a primer capable of hybridizing to each miRNA of miR371, miR372, and miR373 is used as the primer for reverse transcription. A forward primer and a reverse primer capable of amplifying cDNA synthesized from the above miRNA are used as the cDNA amplification primer. Each primer can be designed as appropriate in accordance with the base sequence of each mature miRNA of miR371, miR372, and miR373.

The polymerase can be selected as appropriate from known reverse transcriptases and known DNA polymerases. Examples of the reverse transcriptase include AMV reverse transcriptase and MMLV reverse transcriptase. Examples of the DNA polymerase include Taq polymerase, Pfu polymerase, and Tth polymerase. A reaction solution may be prepared by using a commercial RT-PCR kit or real time RT-PCR kit.

In the present embodiment, when synthesis of cDNA from miRNA and amplification of cDNA are performed in one step, a reaction solution can be prepared in the following manner, for example. A liquid containing miRNA prepared from a liquid phase fraction; a reverse transcription primer capable of hybridizing to miRNA to be measured; a forward primer and a reverse primer capable of amplifying cDNA synthesized from the miRNA; and a reverse transcriptase and a DNA polymerase, are mixed together. A buffer solution and a reagent necessary for nucleic acid amplification reaction such as dNTPs may further be added thereto.

When synthesis of cDNA from miRNA and amplification of cDNA are performed in two steps, a reaction solution can be prepared in the following manner, for example. First, a liquid containing miRNA prepared from a liquid phase fraction; a reverse transcription primer; and a reverse transcriptase are mixed together to prepare a reverse transcription reaction solution. A buffer solution and a reagent necessary for nucleic acid amplification reaction such as dNTPs may further be added thereto. Then, reverse transcription is performed by using the reverse transcription reaction solution. Next, the reverse transcription reaction solution of which the reaction has been completed; a forward primer and a reverse primer capable of amplifying cDNA; and a DNA polymerase are mixed together. Accordingly, a cDNA amplification reaction solution can be obtained. A buffer solution and a reagent necessary for nucleic acid amplification reaction such as dNTPs may further be added thereto.

In the present embodiment, DNA fragments including cDNA of the above miRNA are amplified in the prepared reaction solution, and an amplification product is detected. The amplification reaction can be performed under a known condition appropriate for reverse transcription of miRNA and amplification of cDNA. The amplification reaction can be performed by using a commercial thermal cycler or a commercial real time PCR apparatus.

In this specification, "detecting" includes: qualitatively determining presence or absence of an amplification product; quantitatively determining the amplification product; and semi-quantitatively detecting the existing amount of the amplification product. The semi-quantitative detection means indicating the existing amount of the amplification product in a stepwise manner such as "-", "+", "++" (negative, weakly positive, strongly positive).

The means for detecting the amplification product is not limited in particular, and can be selected as appropriate from known methods. For example, the amplification product may be detected by obtaining optical information such as fluorescence intensity, turbidity, and absorbance from the reaction solution having been subjected to nucleic acid amplification reaction. For example, the amplification product may be detected by measuring a fluorescence intensity by an intercalator method using a fluorescent substance, such as SYBR (trademark) Green, capable of binding to double-stranded DNA. Alternatively, a method using a probe, such as TaqMan (trademark) probe, of which the 5' end is modified with a fluorescent substance and of which the 3' end is modified with a quencher substance is used to detect fluorescence generated from the probe, whereby the amplification product may be detected. Such a probe is designed so as to hybridize to a region different from a region to which the above primer hybridizes in the amplification product to be detected.

For example, when a result that the amplification product had been detected has been obtained, the result indicates that the above-described miRNA is contained in the liquid phase fraction. As described above, the present inventors have found that naive state pluripotent stem cells release miR371, miR372, and miR373 to the outside of the cells. Thus, in the present embodiment, the detection result may be used as an index for existence of naive state pluripotent stem cells in the liquid that contains undifferentiated cells.

In the present embodiment, before the liquid phase fraction is obtained, in the liquid that contains undifferentiated cells, the undifferentiated cells may be induced into naive state pluripotent stem cells. For example, when the undifferentiated cells are primed state pluripotent stem cells, the primed state pluripotent stem cells can be induced into naive state pluripotent stem cells through the above-described gene introduction or culture using the induction medium. In the present embodiment, a reaction solution may be prepared by using a liquid phase fraction collected from a liquid that contains undifferentiated cells having been subjected to induction, and an amplification product derived from at least one miRNA selected from miR371, miR372, and miR373 may be detected. In this case, the detection result can be used in monitoring of the induction state from the primed state type into the naive state type.

Another embodiment of the present invention is a mixed liquid that contains: a liquid phase fraction of a cell culture solution of undifferentiated cells; a reverse transcriptase; a primer capable of hybridizing to at least one miRNA selected from miR371, miR372, and miR373; and dNTPs. The primer capable of hybridizing to at least one miRNA selected from miR371, miR372, and miR373 is at least one selected from: a primer having a base sequence indicated in (1) below on the 3' end side; a primer having a base sequence indicated in (2) below on the 3' end side; a primer having a base sequence indicated in (3) below on the 3' end side; and a primer having a base sequence indicated in (4) below on the 3' end side.

5'-ACACT-3' ··· (1)

5'-GCACT-3' ··· (2)

5'-ACGCT-3' ··· (3)

5'-ACACC-3' ··· (4)

The above-described mixed liquid may further include a DNA polymerase for amplifying cDNA synthesized from miRNA. Details of the reverse transcriptase, the DNA polymerase, and the primer are as described above.

In the following, the present invention is described in more detail by using Examples. However, the present invention is not limited to these Examples.

### [Example]

### Example 1

### (1) Preparation of iPS cells

In Example 1, human iPS cell HPS0063 (201B7 clone) obtained from iPS PORTAL, Inc. was used as iPS cells. HPS0063 cells are primed state iPS cells. When the HPS0063 cells are cultured in an ordinary ES/iPS cell medium, the primed state thereof is maintained. Primed state iPS cells can be induced into naive state iPS cells without gene introduction, by culturing the primed state iPS cells in a naive state iPS cell induction medium such as ReproNaive (trademark). In Example 1, HPS0063 cells were cultured in the procedure indicated below, whereby primed state iPS cells and naive state iPS cells were obtained.

### (1.1) Preparation of primed state iPS cells

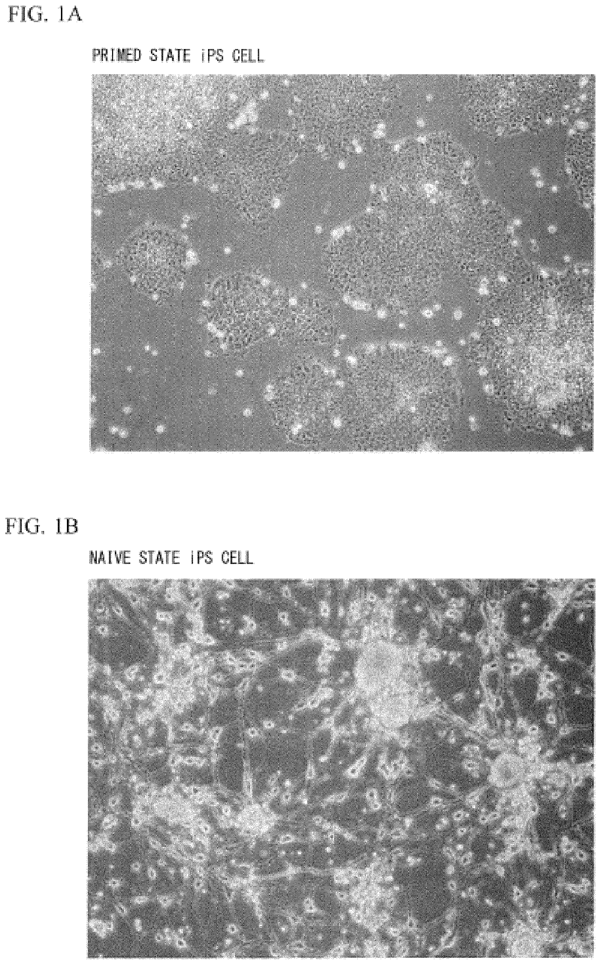

10 mM Y-27632 was added and mixed to a maintaining culture medium Stem Fit (trademark) AK02N (manufactured by Takara Bio Inc.) to obtain a final concentration of 10 µM, whereby a culture medium was prepared. The prepared culture medium (1 mL) was put into a 6-well plate (manufactured by Iwaki) of which the inside was coated with a cell culture substrate iMatrix-511 (manufactured by Nippi Inc.). iPS cells (10⁵ cells/1.0 mL) cultured in advance in the above-described culture medium were added thereto, and the resultant mixture was cultured in a CO₂ incubator (37°C, 5% CO₂) for one day. After the culture, a culture supernatant was collected. FIG. 1A shows a bright field image of the cultured cells.

### (1.2) Preparation of naive state iPS cells

ReproNaive (trademark) Supplement (34.0 µL, manufactured by ReproCELL Inc.), 10 µg/mL LIF (10 µL), and 10 mM Y-27632 (5 µL) were added to ReproNaive (trademark) Basal Medium (5 mL, manufactured by ReproCELL Inc.), to prepare a culture medium. The prepared culture medium (1 mL) was added to a 6-well plate (manufactured by Iwaki) in which SL10 feeder cells were seeded. iPS cells (10⁵ cells/1.0 mL) cultured in advance in the above-described culture medium were added thereto, and the resultant mixture was cultured in a CO₂ incubator (37°C, 5% CO₂) for one day. After the culture, a culture supernatant was collected. FIG. 1B shows a bright field image of the cultured cells.

### (1.3) Preparation of measurement sample

50 µL of the supernatant collected in (1.1) above and 50 µL of the supernatant collect in (1.2) above were used as measurement samples. 50 µL of a medium Essential 8 (manufactured by Gibco) not containing cells and not having been subjected to cell culture was used as a negative control.

### (2) Immunostaining of transcription factor TFE3

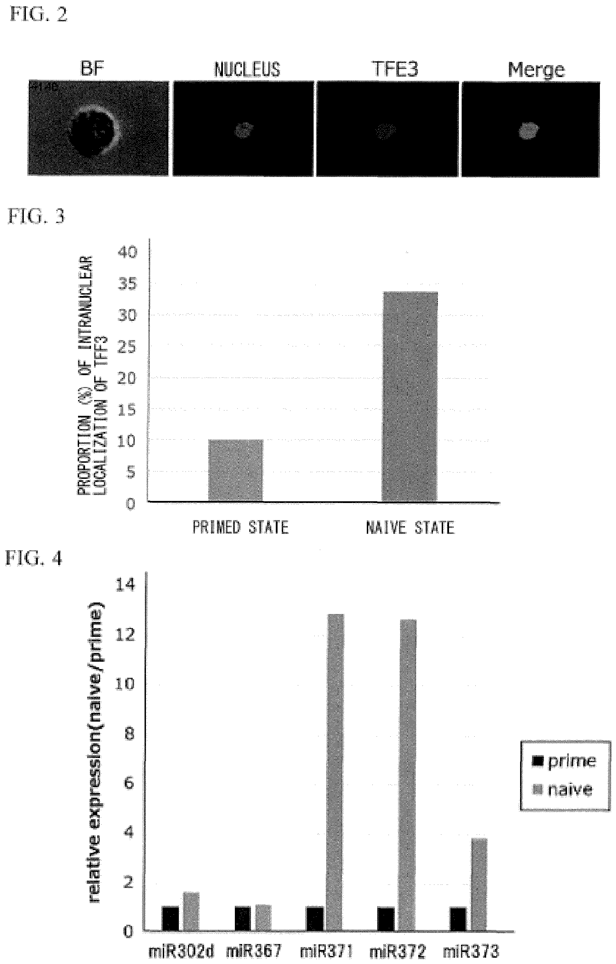

Human iPS cells (201B7 line) were cultured in the same manner as (1.1) above, in a 6-well plate (manufactured by Iwaki) in which SL10 feeder cells were seeded. In addition, the 201B7 line was cultured in the same manner as (1.2) above, in another 6-well plate coated with iMatrix-511 (manufactured by Nippi Inc.). The iPS cells in the well plates were immobilized by a 2% paraformaldehyde-containing PBS. The immobilized iPS cells were washed with PBS, and then subjected to permeabilization using a PBS containing 0.5% saponin and 0.1% BSA. Then, the resultant iPS cells were incubated at room temperature for 30 minutes together with: a solution of Alexa Fluor (trademark) 647 labeled rabbit anti-TFE3 antibody (ab210650, manufactured by Abcam plc.) diluted to 1:50 by a PBS solution containing 0.5% saponin and 0.1% BSA; and a solution of Hoechst (Dojindo) diluted to 1:1000. The resultant iPS cells were washed three times with a PBS containing 0.5% saponin and 0.1% BSA. The iPS cells immunostained in this manner were observed by an imaging-flow cytometer (ImageStreamX MarkII Imaging Flow Cytometer, manufactured by Amnis). FIG. 2 shows an example of a cell having TFE3 localized in the nucleus. In FIG. 2, from the left panel, a bright field image (BF), a nucleus, TFE3, and nucleus/TFE3 (Merge) are shown.

### (3) Counting of iPS cells having TFE3 localized in nuclei

Out of the immunostained cells in the 6-well plate in (2) above, iPS cells that have TFE3 localized in the nuclei were counted, and the proportion thereof was calculated. FIG. 3 shows the proportion of cells having TFE3 localized in the nuclei, for each of the naive state iPS cells and the primed state iPS cells.

### (4) Measurement of miRNA in culture supernatant

Total RNA was collected from each measurement sample obtained in (1) above, by using a High Pure miRNA isolation kit (Roche Diagnostics). The obtained Total RNA (1 µL) was subjected to quantitative RT-PCR, and miR302d, miR367, miR371, miR372, and miR373 were each measured. The quantitative RT-PCR was performed in accordance with the attached protocol, by using TaqMan (trademark) MicroRNA Reverse Transcription Kit, TaqMan (trademark) MicroRNA Assays, TaqMan (trademark) Universal Master Mix II, and Applied Biosystems (trademark) 7500 fast (manufactured by Thermo Fisher Scientific Inc.). FIG. 4 shows, for each miRNA, a relative expression level in the culture supernatant of the naive state iPS cells, with the expression level in the culture supernatant of the primed state iPS cells assumed to be 1.

### (5) Result

As shown in FIG. 1A, under the culture condition maintaining the primed state type, the iPS cells formed flat and large colonies. This is a morphology characteristic to the colony of primed state cells, and it was shown that primed state iPS cells were able to be prepared. As shown in FIG. 1B, under the culture condition of inducing into the naive state type, the iPS cells formed dome-like and small colonies. This is a morphology characteristic to the colony of naive state cells, and it was shown that naive state iPS cells were able to be prepared. As shown in FIG. 3, intranuclear localization of TFE3 was observed at a higher proportion in the prepared naive state iPS cells than in the primed state iPS cells. As also described in NON PATENT LITERATURE 1, TFE3 has characteristics of being localized in the nucleus in the case of naive state iPS cells and being dispersed in the entirety of the cytoplasm in the case of primed state iPS cells. From these, in the present Example, it was confirmed that naive state iPS cells and primed state iPS cells were able to be prepared.

As shown in FIG. 4, it was revealed that the amounts of miR371, miR372, and miR373 are remarkably larger in the culture supernatant of the naive state iPS cells when compared with the culture supernatant of the primed state iPS cells. This shows that, on the basis of measurement values of miR371, miR372, and miR373 contained in the culture supernatant of undifferentiated cells, it is possible to determine which of the naive state and the primed state the undifferentiated cells are in. Meanwhile, with respect to the amounts of miR302-and miR367 in the culture supernatant, there is no difference between the culture supernatant of the primed state iPS cells and the culture supernatant of the naive state iPS cells. Here, miR302 and miR367 are known to have high expression levels in naive state iPS cells. In the result of this quantitative measurement as well, it has been confirmed that the measurement values of miR302 and miR367 were greater than those of miR371, miR372, and miR373. From this, even when the expression level of a miRNA is high in naive state iPS cells, the miRNA having the high expression level is not necessarily released to the outside of the cells, and it cannot be said that the miRNA can be used in the evaluation method of the present embodiment.

## Claims

1. A method for evaluating a state of undifferentiated cells, the method comprising the steps of:
measuring at least one miRNA selected from miR371, miR372, and miR373 in a liquid phase fraction of a liquid comprising undifferentiated cells; and
evaluating a state of the undifferentiated cells on the basis of a measurement value of the miRNA.

2. The method according to claim 1, wherein the undifferentiated cells are pluripotent stem cells.

3. The method according to claim 2, wherein the pluripotent stem cells are iPS cells, ES cells, ntES cells, or EG cells.

4. The method according to claim 2 or 3, wherein the measurement value of the miRNA is an index for existence of naive state pluripotent stem cells in the liquid.

5. The method according to any one of claims 2 to 4, comprising determining that the undifferentiated cells are naive state pluripotent stem cells when the measurement value of the miRNA is greater than or equal to a first threshold,.

6. The method according to any one of claims 2 to 5, comprising determining that the undifferentiated cells are primed state pluripotent stem cells when the measurement value of the miRNA is smaller than a second threshold.

7. The method according to any one of claims 1 to 6, wherein the liquid comprising undifferentiated cells is a cell culture solution of the undifferentiated cells.

8. The method according to any one of claims 1 to 7, wherein the miR371 is miR371a or miR371b.

9. The method according to any one of claims 1 to 8, wherein the measurement value of the miRNA is a miR371a measurement value, a miR371b measurement value, a miR372 measurement value, a miR373 measurement value, or a sum of at least two of the measurement values.

10. The method according to any one of claims 1 to 9, wherein the measurement value of the miRNA is measured according to a quantitative RT-PCR method.

11. A reagent kit for use in the method according to any one of claims 1 to 10, the reagent kit comprising at least one primer capable of hybridizing to at least one miRNA selected from miR371, miR372, and miR373.

12. A detection method of an amplification product, the detection method comprising the steps of:
obtaining a liquid phase fraction of a liquid comprising undifferentiated cells;
mixing at least one miRNA selected from miR371, miR372, and miR373 contained in the liquid phase fraction, a primer, and a polymerase together, to prepare a reaction solution; and
amplifying DNA fragments comprising cDNA of the miRNA in the reaction solution and detecting an amplification product.

13. The detection method according to claim 12, wherein
a result of the detection serves as an index for existence of naive state pluripotent stem cells in the liquid.

14. The detection method according to claim 12 or 13, further comprising the step of inducing the undifferentiated cells into naive state pluripotent stem cells in the liquid before obtaining the liquid phase fraction.

15. A method for monitoring an induction operation of undifferentiated cells, the method comprising:
obtaining a liquid phase fraction of a liquid comprising undifferentiated cells, before an induction operation from primed state undifferentiated cells into naive state undifferentiated cells, and obtaining a first measurement value of at least one miRNA selected from miR371, miR372, and miR373 in the liquid phase fraction;
obtaining a liquid phase fraction of the liquid comprising undifferentiated cells, during the induction operation from primed state undifferentiated cells into naive state undifferentiated cells, and obtaining a second measurement value of at least one miRNA selected from miR371, miR372, and miR373 in the liquid phase fraction; and
comparing the first measurement value and the second measurement value with each other, thereby monitoring the induction operation of the undifferentiated cells.

16. A method for monitoring an induction operation of undifferentiated cells, the method comprising:
obtaining a liquid phase fraction of a liquid comprising undifferentiated cells, at a first time point during an induction operation from primed state undifferentiated cells into naive state undifferentiated cells, and obtaining a first measurement value of at least one miRNA selected from miR371, miR372, and miR373 in the liquid phase fraction;
obtaining a liquid phase fraction of the liquid comprising undifferentiated cells, at a second time point during the induction operation from primed state undifferentiated cells into naive state undifferentiated cells, and obtaining a second measurement value of at least one miRNA selected from miR371, miR372, and miR373 in the liquid phase fraction; and
comparing the first measurement value and the second measurement value with each other, thereby monitoring the induction operation of the undifferentiated cells.

17. A reagent kit comprising at least one primer selected from: a primer comprising a base sequence indicated in (1) below on a 3' end side; a primer comprising a base sequence indicated in (2) below on a 3' end side; a primer comprising a base sequence indicated in (3) below on a 3' end side; and a primer comprising a base sequence indicated in (4) below on a 3' end side.
5'-ACACT-3' ··· (1)
5'-GCACT-3' ··· (2)
5'-ACGCT-3' ··· (3)
5'-ACACC-3' ··· (4)

18. A mixed liquid comprising: a liquid phase fraction of a cell culture solution of undifferentiated cells; a reverse transcriptase; a primer capable of hybridizing to at least one miRNA selected from miR371, miR372, and miR373; and dNTPs.

19. The mixed liquid according to claim 18, wherein
the primer is at least one selected from: a primer comprising a base sequence indicated in (1) below on a 3' end side; a primer comprising a base sequence indicated in (2) below on a 3' end side; a primer comprising a base sequence indicated in (3) below on a 3' end side; and a primer comprising a base sequence indicated in (4) below on a 3' end side.
5'-ACACT-3' ··· (1)
5'-GCACT-3' ··· (2)
5'-ACGCT-3' ··· (3)
5'-ACACC-3' ··· (4)

20. The mixed liquid according to claim 18 or 19, wherein
the liquid phase fraction includes at least one miRNA selected from miR371, miR372, and miR373.


SEQUENCE LISTING

<110>  Sysmex Corporation

<120>  A method for evaluating status of undifferentiated cells and
       use thereof

<130>  18066WO1

<160>  4

<170>  PatentIn version 3.5

<210>  1
<211>  23
<212>  RNA
<213>  Homo sapiens

<400>  1
aagugccgcc aucuuuugag ugu                                              23


<210>  2
<211>  23
<212>  RNA
<213>  Homo sapiens

<400>  2
aagugccccc acaguuugag ugc                                              23


<210>  3
<211>  23
<212>  RNA
<213>  Homo sapiens

<400>  3
aaagugcugc gacauuugag cgu                                              23


<210>  4
<211>  23
<212>  RNA
<213>  Homo sapiens

<400>  4
gaagugcuuc gauuuugggg ugu                                              23